(19) Europäisches Patentamt
European Patent Office
Office européen des brevets

(11) EP 0 913 143 A2

(12) **EUROPÄISCHE PATENTANMELDUNG**

(43) Veröffentlichungstag:
**06.05.1999 Patentblatt 1999/18**

(51) Int. Cl.$^6$: **A61K 7/06**

(21) Anmeldenummer: **98111949.8**

(22) Anmeldetag: **29.06.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **24.07.1997 DE 19731907**

(71) Anmelder:
**BASF AKTIENGESELLSCHAFT**
**67056 Ludwigshafen (DE)**

(72) Erfinder:
- **Zeitz, Katrin, Dr.**
  **67067 Ludwigshafen (DE)**
- **Hössel, Peter, Dr.**
  **67105 Schifferstadt (DE)**
- **Dieing, Rinhold, Dr.**
  **67105 Schifferstadt (DE)**
- **Sanner, Axel, Dr.**
  **67227 Frankenthal (DE)**

(54) **Vernetzte kationische Copolymere mit N-Vinylimidazolen**

(57) Verwendung von Polymeren, die erhältlich sind durch

(i) radikalisch initiierte Copolymerisation von Monomergemischen aus

(a) 1 bis 99,99 Gew.-% eines N-Vinylimidazols oder eines quaternisierten N-Vinylimidazols,
(b) 0 bis 98,99 Gew.-% eines neutralen oder basischen wasserlöslichen Monomeren,
(c) 0 - 49,99 Gew.-% einer ungesättigten Säure oder eines ungesättigten Anhydrids
(d) 0 bis 50 Gew.-% eines weiteren radikalisch copolymerisierbaren Monomeren und
(e) 0,01 bis 10 Gew.-% eines bi- oder mehrfunktionellen radikalisch copolymerisierbaren Monomeren, und

(ii) und anschließende Quaternisierung oder Protonierung des Polymeren für den Fall, daß als Monomeres (a) ein nicht quaternisiertes N-Vinylimidazol eingesetzt wird,

als konditionierende und haarfestigende Gelbildner in kosmetischen Zusammensetzungen.

EP 0 913 143 A2

**Beschreibung**

Die vorliegende Erfindung betrifft die Verwendung von Polymeren als Wirkstoffe in kosmetischen Zubereitungen, bevorzugt in haarkosmetischen Zubereitungen.

Als Konditioniermittel in kosmetischen Formulierungen werden kationische Polymere eingesetzt. Sie bewirken in erster Linie eine Verbesserung der Naßkämmbarkeit des Haares. Außerdem verhindern kationische Polymere die elektrostatische Aufladung des Haares. Der Einsatz von kationischen Polymeren in der Haarkosmetik wird beschrieben in "The History of Hair Care" , Cosmetics & Toiletries, Vol. 103, Dec. 1988, S. 23.

Marktübliche Gelbildner bestehen aus anvernetzten Polyacrylsäuren, die bei Neutralisation Gele ausbilden. Aufgrund ihrer anionischen Natur zeigen diese Polymere eine Unverträglichkeit mit kationischen Polymeren, so daß polykationische Konditioniermittel, wenn überhaupt, nur in kleinen Mengen mit solchen anionischen Gelen kombiniert werden können. Die konditionierende Wirkung dieser kosmetischen Formulierungen ist deshalb gering.

Polymere mit quaternierten N-Vinylimidazolen sind bekannt. So wird in EP 246580 die Verwendung von Homo- und Copolymeren von 3-Methy-1-vinylimidazoliumchloriden u. a. als Haarkonditioniermittel beschrieben.

EP 544158 und US 4 859 756 beanspruchen die Verwendung von Homo- und Copolymeren von chloridfreien, quaternisierten N-Vinylimidazolen in kosmetischen Zubereitungen.

Aus EP 715843 ist die Verwendung von Copolymeren aus einem quaternierten N-Vinylimidazol, N-Vinylcaprolactam und N-Vinylpyrrolidon sowie optional einem weiteren Comonomer in kosmetischen Zubereitungen bekannt.

In keinem der genannten Patente ist die Verwendung von vernetzten Polymeren beschrieben.

DE 3209224 beschreibt die Herstellung von vernetzten Polymerisaten auf Basis N-Vinylpyrrolidon und (quaternisiertem) N-Vinylimidazol. Diese Polymerisate werden für die Verwendung als Adsorbentien und Ionenaustauscher beansprucht.

Vernetzte, agglomerierte Vinylimidazol-Copolymerisate werden in WO96/26229 als Farbstoffübertragungsinhibitoren genannt. Sie sind hochvernetzt, wasserunlöslich, wenig quellbar und daher nicht geeignet als Konditioniermittel oder Gelbildner in kosmetischen Formulierungen.

Aus US 4 058 491 sind vernetzte kationische Hydrogele aus N-Vinylimidazol oder N-Vinylpyrrolidon und einem quaternierten basischen Acrylat sowie weiteren Comonomeren bekannt. Diese Gele werden zur Komplexierung und kontrollierten Freisetzung anionischer Wirksubstanzen vorgeschlagen.

WO 96/37525 beschreibt die Herstellung von vernetzten Copolymeren aus u. a. N-Vinylpyrrolidon und quaternierten Vinylimidazolen in Gegenwart von Polymerisationsreglern und ihre Verwendung insbesondere in Waschmitteln. Als Gelbildner sind die Verbindungen ungeeignet.

DE 4213971 beschreibt Copolymerisate aus einer ungesättigten Carbonsäure, quaternisiertem Vinylimidazol und optional weiteren Monomeren und einem Vernetzer. Die Polymere werden als Verdickungs- und Dispergiermittel vorgeschlagen.

US 4 806 345 nennt vernetzte kationische Verdicker für kosmetische Formulierungen aus quaterniertem Dimethylaminoethylmethacrylat und Acrylamid.

Aus WO 93/25595 sind vernetzte Copolymere aus einem N-Vinyllactam, einem quaternisierten Aminoalkylacrylamid oder Aminoalkyl(meth)acrylat sowie optional einer ungesättigten Carbonsäure bekannt. Die Copolymere werden als Verdicker für kosmetische Formulierungen beschrieben.

Die Verfahrensweise des Verdickens durch Protonierung eines wasserlöslichen, vernetzten Aminoalkyl(meth)acrylat wird in EP 624617 und EP 27850 beschrieben.

Die Anwendung von Copolymeren mit einem Aminoalkyl(meth)acrylat in der Kosmetik ist in EP 0671157 beschrieben. Die dort erwähnten Polymere werden aber ausschließlich für die gemeinsame Anwendung mit Festiger- oder Conditionerpolymeren verwendet.

Aufgabe der vorliegenden Erfindung war es, Wirkstoffe für kosmetische Formulierungen bereitzustellen, die alle oder möglichst viele der folgenden Eigenschaften erfüllen:

gute Gel-bildende Wirkung
gute haarfestigende Wirkung
gute konditionierende Wirkung
gute Hautverträglichkeit
gute Verträglichkeit mit anderen Bestandteilen von kosmetischen Formulierungen.

Gegenstand der Erfindung ist die Verwendung von Polymeren, die erhältlich sind durch

(i) radikalisch initiierte Copolymerisation von Monomergemischen aus

(a) 1 bis 99,99 Gew.-% eines N-Vinylimidazols oder eines quaternisierten N-Vinylimidazols,

(b) 0 bis 98,99 Gew.-% eines neutralen oder basischen wasserlöslichen Monomeren,
(c ) 0 - 49,99 Gew.-% einer ungesättigten Säure oder eines ungesättigten Anhydrids,
(d ) 0 bis 50 Gew.-% eines weiteren radikalisch copolymerisierbaren Monomeren und
(e) 0,01 bis 10 Gew-% eines bi- oder mehrfunktionellen radikalisch copolymerisierbaren Monomeren und

(ii) und anschließende Quaternisierung oder Protonierung des Polymeren für den Fall, daß als Monomeres (a) ein nicht quaternisiertes N-Vinylimidazol eingesetzt wird,

als konditionierende und haarfestigende Gelbildner in kosmetischen Zusammensetzungen.

Als Monomere (a) geeignete N-Vinylimidazole sind die N-Vinylimidazol-Derivate der allgemeinen Formel I, worin $R^1$ bis $R^3$ für Wasserstoff, $C_1$ - $C_4$ -Alkyl oder Phenyl steht.

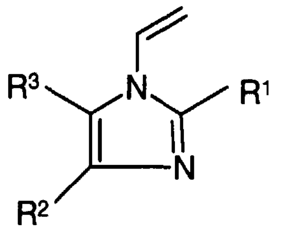

(I)

Zur Quaternisierung des Vinylimidazols eignen sich beispielsweise Alkylhalogenide mit 1 bis 22 C-Atomen in der Alkylgruppe, z. B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der Vinylimidazole kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternierung der basischen Monomere der allgemeinen Formel (I) kann auch mit Alkylenoxiden wie Ethylenoxid oder Propylenoxid in Gegenwart von Säuren durchgeführt werden.

Ganz besonders bevorzugte Monomere der Gruppe (a) sind 3-Methyl-1-vinylimidazoliumchlorid und -methosulfat.

Die Quaternisierung des Monomeren oder eines Polymeren mit einem der genannten Quaternisierungsmittel kann nach allgemein bekannten Methoden erfolgen.

Die Polymere enthalten 1- 99,99, bevorzugt 2-95 und besonders bevorzugt 10 bis 70 Gew.-% Monomer (a).

Geeignete neutrale oder basische wasserlösliche Monomere (b) sind N-Vinyllactame, z.B. N-Vinylpiperidon, N-Vinylpyrrolidon und N-Vinylcaprolactam, N-Vinylacetamid, N-Methyl-N-vinylacetamid, Acrylamid, Methacrylamid, N,N-Dimethylacrylamid, N-Methylolmethacrylamid, N-Vinyloxazolidon, N-Vinyltriazol, Hydroxyalkyl(meth)acrylate, z.B. Hydroxyethyl(meth)acrylat und Hydroxypropyl(meth)acrylate oder Alkylethylenglykol(meth)acrylate mit 1 bis 50 Ethylenglykoleinheiten im Molekül.

Außerdem eignen sich N-Vinylimidazole der allgemeinen Formel (I), worin $R^1$ bis $R^3$ für Wasserstoff, $C_1$ - $C_4$-Alkyl oder Phenyl steht, Diallylamine der allgemeinen Formel (II) sowie Dialkylaminoalkyl(meth)acrylate und Dialkylaminoalkyl(meth)acrylamide der allgemeinen Formel (III), z.B. Dimethylaminoethylmethacrylat oder Dimethylaminopropylmethacrylamid.

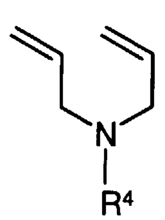

(II)

Bevorzugt werden als Monomere (b) N-Vinyllactame eingesetzt. Ganz besonders bevorzugt ist N-Vinylpyrrolidon.

Die Polymere enthalten 0-98, bevorzugt 5-80 und besonders bevorzugt 10 bis 70 Gew.-% Monomer (b).

Als Monomere (c) eignen sich ungesättigte Carbonsäuren und ungesättigte Anhydride,bevorzugt Acrylsäure, Methacrylsäure, Crotonsäure, Itaconsäure, Maleinsäure, Fumarsäure oder ihre entsprechenden Anhydride sowie ungesättigte Sulfonsäuren,bevorzugt Acrylamidomethylpropansulfonsäure.

Die Polymere enthalten 0-50, bevorzugt 0-40 und besonders bevorzugt 0 bis 25 Gew.-% Monomer (c).

Als Monomere (d) eignen sich besonders $C_1$ bis $C_{18}$-Alkylester der (Meth)acrylsäure, z. B. Methyl(meth)acrylat, Ethyl(meth)acrylat, tert.-Butyl(meth)acrylat, Isobutyl(meth)acrylat, n-Butyl(meth)acrylat und Acrylamide wie N-tert.-Butylacrylamid oder N-tert.-Octylacrylamid. Ferner eignen sich Carbonsäurevinylester, z.B. Vinylacetat oder Vinylpropionat.

Die Polymere enthalten 0-50 bevorzugt 0-40 und besonders bevorzugt 0 bis 25 Gew.-% Monomer (d).

Als Monomere (e) (Vernetzer) sind Verbindungen geeignet mit mindestens 2 ethylenisch ungesättigten, nichtkonjugierten Doppelbindungen im Molekül.

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexan-diol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C3- bis C6-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet sind außerdem geradkettig oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 - 20 000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Weitere geeignete Vernetzer sind Divinyldioxan, Tetraallylsilan oder Tetravinylsilan.

Vorzugsweise werden solche Vernetzer eingesetzt, die in der Monomermischung löslich sind.

Besonders bevorzugt eingesetzte Vernetzer sind beispielsweise Methylenbisacrylamid, Triallylamin und Triallylalkylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Methylenbisacrylamid, N,N'-Divinylethylenharnstoff und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

Der Vernetzer ist vorzugsweise im Reaktionsmedium löslich. Ist die Löslichkeit des Vernetzers im Reaktionsmedium gering, so kann er in einem Monomeren oder in einer Monomerenmischung gelöst werden oder aber in einem Lösungsmittel gelöst zudosiert werden, das sich mit dem Reaktionsmedium mischt.

Wird in wässeriger Lösung polymerisiert, werden bevorzugt wasserlösliche Vernetzer eingesetzt, z.B. Divinylethylenharnstoff, Triallylamin, Triallylmonoalkylammoniumsalze oder Polyethylenglykoldi(meth)acrylate.

Durch den Gehalt an Vernetzter kann die Lösungsviskosität der erfindungsgemäßen Polymere in weitem Maße beeinflußt werden.

Die Polymere enthalten 0,01-10, bevorzugt 0,05-8 und besonders bevorzugt 0,1-5 Gew.-% Monomer (e).

Durch Variation des Vernetzergehaltes können auf Basis der erfindungsgemäßen Polymere kationische Gele hergestellt werden, die ohne Zusatz eines weiteren Konditioniermittels konditionierend wirken.

Die Monomere (a) bis (e) können jeweils einzeln oder im Gemisch mit anderen Monomeren der gleichen Gruppe eingesetzt werden.

Die Herstellung der Polymerisate kann nach den an sich bekannten Verfahren der radikalisch initiierten Polymerisation, z. B. durch Lösungspolymerisation, Fällungspolymerisation, Umgekehrte Suspensionspolymerisation oder Inverse Emulsionspolymerisation erfolgen.

Als Initiatoren für die radikalische Polymerisation können die hierfür üblichen wasserlöslichen und wasserunlöslichen Peroxo- und/oder Azo-Verbindungen eingesetzt werden, beispielsweise Alkali- oder Ammoniumperoxidisulfate, Dibenzoylperoxid, tert.-Butylperpivalat, tert.-Butyl-per-2-ethylhexanoat, Di-tert.-butylperoxid, Text.-Butylhydroperoxid, Azo-bis-isobutyronitril, Azobis-(2-amidinopropan)dihydrochlorid oder 2,2'-Azo-bis-(2-methylbutyronitril). Geeignet sind auch Initiatormischungen oder Redox-Initiator Systeme, wie z.B. Ascorbinsäure/Eisen(II)sulfat / Natriumperoxodisulfat, tert.-Butylhydroperoxid /Natriumdisulfit, tert.-Butylhydroperoxid/Natriumhydroxymethansulfinat. Die Initiatoren können in den üblichen Mengen eingesetzt werden, beispielsweise 0,05 bis 5 Gew.-%, bezogen auf die Menge der zu polymerisierenden Monomeren.

Die erfindungsgemäß verwendeten vernetzten Polymere besitzen eine sehr gute konditionierende und festigende Wirkung in haarkosmetischen Anwendungen. Kationische Gele auf Basis der erfindungsgemäßen Polymere zeigen eine so gute konditionierende Wirkung, daß ohne Zusatz eines weiteren Konditioniermittels konditionierende Gel-Formulierungen hergestellt werden können.

Falls gewünscht, können auch weitere kationische Konditioniermittel zugesetzt werden, da aufgrund der kationischen Natur der Gele keine Unverträglichkeit mit kationischen Polymeren besteht.

Gegenüber Polymeren mit z.B. quaternisierten Aminoalkyl(meth)acrylaten zeichnen sich die erfindungsgemäßen Polymere auf Basis kationischer N-Vinylimidazole durch ihre Hydrolysestabilität und die höhere Ladungsdichte aus. Die besondere Anwendungsmöglichkeit für Haargele ergibt sich aus ihrer gleichzeitig festigenden und konditionierenden Wirkung.

Die Monomere (a) können entweder in quaternierter Form als Monomere eingesetzt werden oder nicht-quaterniert polymerisiert werden, wobei man im letzteren Fall das erhaltene Copolymer entweder quaterniert oder protoniert.

Zur Protonierung eignen sich beispielsweise Mineralsäuren wie HCl, $H_2SO_4$, $H_3PO_4$, sowie Monocarbonsäuren z.B. Ameisensäure und Essigsäure, Dicarbonsäuren und mehrfunktionelle Carbonsäuren, z.B. Oxalsäure und Zitronensäure sowie alle anderen protonenabgebenden Verbindungen und Substanzen, die in der Lage sind das entsprechende Vinylimidazol zu protonieren. Insbesondere eignen sich wasserlösliche Säuren zur Protonierung.

Die Protonierung des Polymers kann entweder im Anschluß an die Polymerisation erfolgen oder bei der Formulierung der kosmetischen Zusammenstellung, bei der in der Regel ein physiologisch verträglicher pH-Wert eingestellt wird.

Unter Protonierung ist zu verstehen, daß mindestens ein Teil der protonierbaren Gruppen des Polymers, bevorzugt 20- 100 %, protoniert wird, so daß eine kationische Gesamtladung des Polymers resultiert.

Ein weiterer Gegenstand der Erfindung sind für die Haarkosmetik geeignete Zusammensetzungen, insbesondere Haargele, Haarstylinggele, Wet-Look-Gele, die eines oder mehrere der oben beschriebenen Polymere enthalten.

Beispiel 1:

Gelpolymerisation:

In einem Laborkneter werden 560 g Wasser, 320 g Vinylpyrrolidon 160g Vinylimidazolium-methosulfat, 0,625 g 2,2'-Azobis(2-amidinopropan)dihydrochlorid und 1,2 g Tripropylenglycoldiacrylat mit Stickstoff gespült und dann auf

70°C aufgeheizt.

Die Polymerisation ist nach 1 h beendet. Nach nochmaliger Initiatorzugabe wird noch 6 h bei 70°C nachpolymerisiert, das Polymerisat ausgetragen, getrocknet und gemahlen.

Beispiel 2:

Umgekehrte Suspension

Ein Reaktionsgefäß mit 800 g Cyclohexan, 5 g Sorbitanmonooleat, 5 g Hypermer B246 (ICI) werden auf 60°C erwärmt, der Zulauf, bestehend aus 100 g Vinylimidazolium-Methosulfat, 100 g Vinylpyrrolidon, 0,33 g 2,2'-Azobis(2-amidinopropan)-dihydrochlorid, 140 g Wasser und 0,25 g Tripropylenglycoldiacrylat, werden innerhalb 1h zugegeben. Es wird 6 h polymerisiert, das Wasser azeotrop abdestilliert, das Polymerisat abfiltriert und getrocknet.

Beispiel 3:

Inverse Emulsion:

Ein Reaktionsgefäß mit 800 g Cyclohexan, 5 g Sorbitanmonooleat, 5 g Hypermer B246 (ICI) und 1 g 2,2'-Azobis(2,4-dimethylvaleronitrile) werden auf 65°C erwärmt, der Zulauf, bestehend aus 100 g Vinylimidazol, 100 g Vinylpyrrolidon, 140 g Wasser und 0,25 g Tripropylenglycoldiacrylat, werden innerhalb 20 min zugegeben. Es wird 6 h polymerisiert, dann 200 g Cyclohexan zugesetzt, das Wasser azeotrop abdestilliert, das Polymerisat abfiltriert und getrocknet.

Verwendung der Polymere in der Haarkosmetik

Die erfindungsgemäßen Gelbildner besitzen eine Fließgrenze, Festigerwirkung, Konditionierwirkung und sind klar formulierbar (siehe Tabelle 1).

Der Vorteil der Fließgrenze zeigt sich bei der Applikation. Nach dem Ausdrücken aus der Tube "steht" das Gel in der Hand und kann trotzdem leicht ins Haar verteilt werden.

Formulierungen (pH-Wert 2 bis 9; der bevorzugte pH-Wert beträgt 5 bis 7):

Beispiel 4

| | |
|---|---|
| 1.5 % | Polymerwirkstoff aus Beispiel 1 |
| ad 100 % | Wasser |
| q.s. | Neutralisationsmittel |

Beispiel 5 (Vergleichsbeispiele)

5

| | |
|---|---|
| 0.5 % | Carbomer |
| ad 100 % | Wasser |
| q.s. | Neutralisationsmittel, z.B. HCl |

6

| | |
|---|---|
| 0.5 % | Carbomer |
| 3.0 % | PVP/VA- Copolymer |
| q.s. | Neutralisationsmittel, z.B. NaOH |

7

| | |
|---|---|
| 0.5 % | Carbomer |

| | |
|---|---|
| 1.0 % | Polyquaternium-11 (gerechnet 100 % als Polymer wirkstoff) |
| 2.0 % | PVP/VA-Copolymer |
| ad 100 % | Wasser |
| q.s. | Neutralisationsmittel, z.B. NaOH |

Tabelle 1: Vorteil der Gelformulierungen mit den erfindungsgemäßen Gelbildnern im Vergleich zu marktüblichen Gelformulierungen mit Kombinationen von Polymeren

| Gelformulierung | Kämmkraftabnahme in % | Festigerwirkung | Aussehen | Viskosität, Fließgrenze |
|---|---|---|---|---|
| 4 | 65 % | sehr gut | klar/glatt | 26000 mPas |
| 5 | 0 % | schwach | klar/glatt | 15000 mPas |
| 6 | 10 % | mäßig | klar/glatt | 23000 mPas |
| 7 | 64 % | sehr gut | trüb/narbig | 27000 mPas |

Testmethoden:

a) Kämmkraftabnahme

An einer Zug/Druck-Prüfmaschine wird die Kämmbarkeit bestimmt, welche notwendig ist, einen Kamm durch eine Haartresse zu ziehen. Die Kämmkraftabnahme wird wie folgt berechnet (je größer der Wert, desto besser die Konditionierwirkung).

$$\% \text{ Kämmkraftabnahme} = 100\ (1 - Ay/Ao)$$

Ay = Kämmarbeit nach Behandlung mit Testshampoo (siehe Beispiele)
Ao = Kämmarbeit nach Behandlung mit Shampoo ohne Polymer (Blindwert)

b) Festigerwirkung

Subjektive Beurteilung an einer mit der Gelformulierung behandelten Haarsträhne nach dem Trocknen des Polymerfilmes.

c) Aussehen

Beurteilt wird die Klarheit des Geles (klar-trüb) und die Struktur nach Aufstreichen auf eine Glasplatte (glattnarbig). Optimale Gelformulierungen haben eine glatte Struktur und sind wasserklar.

## Patentansprüche

1. Verwendung von Polymeren, die erhältlich sind durch

   (i) radikalisch initiierte Copolymerisation von Monomergemischen aus

   (a) 1 bis 99,99 Gew.-% eines N-Vinylimidazols oder eines quaternisierten N-Vinylimidazols,
   (b) 0 bis 98,99 Gew.-% eines neutralen oder basischen wasserlöslichen Monomeren,
   (c) 0 - 49,99 Gew.-% einer ungesättigten Säure oder eines ungesättigten Anhydrids
   (d) 0 bis 50 Gew.-% eines weiteren radikalisch copolymerisierbaren Monomeren und
   (e) 0,01 bis 10 Gew.-% eines bi- oder mehrfunktionellen radikalisch copolymerisierbaren Monomeren, und

   (ii) und anschließende Quaternisierung oder Protonierung des Polymeren für den Fall, daß als Monomeres (a) ein nicht quaternisiertes N-Vinylimidazol eingesetzt wird,

als konditionierende und haarfestigende Gelbildner in kosmetischen Zusammensetzungen.

**2.** Verwendung nach Anspruch 1, dadurch gekennzeichnet, daß die Protonierung gemäß (ii) bei der Formulierung der kosmetischen Zusammensetzung erfolgt.

**3.** Verwendung nach Anspruch 1 und 2, dadurch gekennzeichnet, daß als Monomer (b) ein Vinyllactam verwendet wird.

**4.** Verwendung nach Anspruch 3, dadurch gekennzeichnet, daß Monomer (b) in Mengen von 30-70 Gew.-% verwendet wird.

**5.** Für die Haarkosmetik geeignete Zusammensetzung enthaltend ein Polymer gemäß Anspruch 1-4.

**6.** Haargele, enthaltend ein Polymer gemäß Anspruch 1-4.